# EUROPÄISCHE PATENTSCHRIFT

(11) **EP 0 705 089 B1**
(45) Veröffentlichungstag und Bekanntmachung des Hinweises auf die Patenterteilung: **14.05.1997**
(21) Anmeldenummer: 94918851.0
(22) Anmeldetag: 08.06.1994
(51) Int. Cl.: A61F 13/42

(54) **WEGWERFWINDEL**
DISPOSABLE DIAPER
COUCHE JETABLE

(30) Priorität: 21.06.1993 DE 9309199 U
(43) Veröffentlichungstag der Anmeldung: 10.04.1996
(73) Patentinhaber: Paul Hartmann Aktiengesellschaft, D-89522 Heidenheim (DE)
(72) Erfinder: GREINER, Jean, F-67000 Strasbourg (FR); MALOWANIEC, Krzysztof, D., D-89522 Heidenheim (DE)
(74) Vertreter: Becker, Maria, Dipl.-Phys.
(86) Internationale Anmeldenummer: EP9401862
(87) Internationale Veröffentlichungsnummer: WO9500099

(56) Entgegenhaltungen:
- DE-A- 2 031 104
- FR-A- 2 575 905
- US-A- 3 952 746
- US-A- 4 022 211
- US-A- 4 681 576
- US-A- 4 705 513

## Beschreibung

Die Erfindung betrifft eine Wegwerfwindel in einer im Oberbegriff des Anspruches 1 erläuterten Ausbildung.

Es sind bereits Wegwerfwindeln bekannt, deren Saugkörper Nässeanzeigemittel zugeordnet sind, um ohne Öffnen der vom Benutzer getragenen Windel erkennen zu können, ob der Grad der Einnässung einen Windelwechsel erforderlich macht.

So offenbart die US-A-4022211 eine Windelausführung, deren Saugkörper ein Träger mit auf diesem angeordneten Nässeindikatoren zugeordnet ist. Die Nässeindikatoren können hierbei musterartig oder in loser Verteilung vorgesehen sein. Sie sind bei nicht benutzter Windel sichtbar und werden in eingenässten Bereichen des Saugkörpers unsichtbar bzw. so verändert, dass dieser Bereich von noch trockenen Windelbereichen abgegrenzt wird.

Des weiteren ist aus der DE-A-2031104, Fig. 2, eine Wegwerfwindel bekannt, die an der Aussenseite des vorderen Windelendrandes mehrere nebeneinander liegende, verfärbbare Indikatoren aufweist, die über jeweils ein streifenförmiges Element aus flüssigkeitsleitendem Werkstoff mit dem Nässungsbereich verbunden sind.

Diese streifenförmigen Leitelemente haben unterschiedliche Längen, so dass im Falle des Einnässens die Anzahl der verfärbten Indikatoren ein Maß für die Ausdehnung des sich bildenden Nässungsherdes darstellt.

Schliesslich ist aus der FR-A-2575905 auch bereits eine Wegwerfwindel in einer Ausbildung gemäss dem Oberbegriff des Anspruches 1 bekannt. Bei ihr sind zwischen Wäscheschutzfolie und Saugkörper im Abstand zueinander parallel und in Windellängsrichtung verlaufende Nässeanzeigestreifen eingebracht, die eingenässte Windelbereiche sichtbar machen.

Die Nässeanzeigestreifen bilden zwei jeweils zwei eingefärbte Streifen umfassende Streifengruppen, die parallel zur Längssymmetrieachse der Windel verlaufen und sich über die gesamte Länge des Saugkörpers erstrecken.

Die Nässeanzeigestreifen betehen aus einem entsprechend teuren, eingefärbten Polymermaterial, das unter Einwirkung von Nässe aufquillt, wodurch sich der Streifenquerschnitt entlang von Teilbereichen der Streifenlänge ausgehend vom mittleren Streifenbereich entsprechend verändert.

Der Erfindung liegt die Aufgabe zugrunde, für Wegwerfwindeln eine Streifenanordnung anzugeben, die im Vergleich zur bekannten Windelausführung zur optimalen Anzeige eingenässter Windelbereiche mit wesentlich geringerer Länge der Nässeanzeigestreifen auskommt, so dass sich bei maximalem Einnässen nicht reagierende und damit überflüssige Streifenabschnitte auf ein Minimum beschränken lassen.

Zur Lösung dieser Aufgabe dienen, ausgehend von einer Wegwerfwindel gemäss dem Oberbegriff des Anspruches 1, dessen kennzeichnenden Anspruchsmerkmale.

Die erfindungsgemässe Plazierung der Nässeanzeigestreifen bietet somit den Vorteil einer maximalen Nutzung von Streifenmaterial in dem Windelbereich, der tatsächlich eingenässt werden kann.

Die Merkmale der Ansprüche 2 - 5 ermöglichen verschiedene, vorteilhafte Ausgestaltungen der Erfindung.

In der Zeichnung sind Ausführungsbeispiele der Erfindung schematisch dargestellt. Es zeigen:

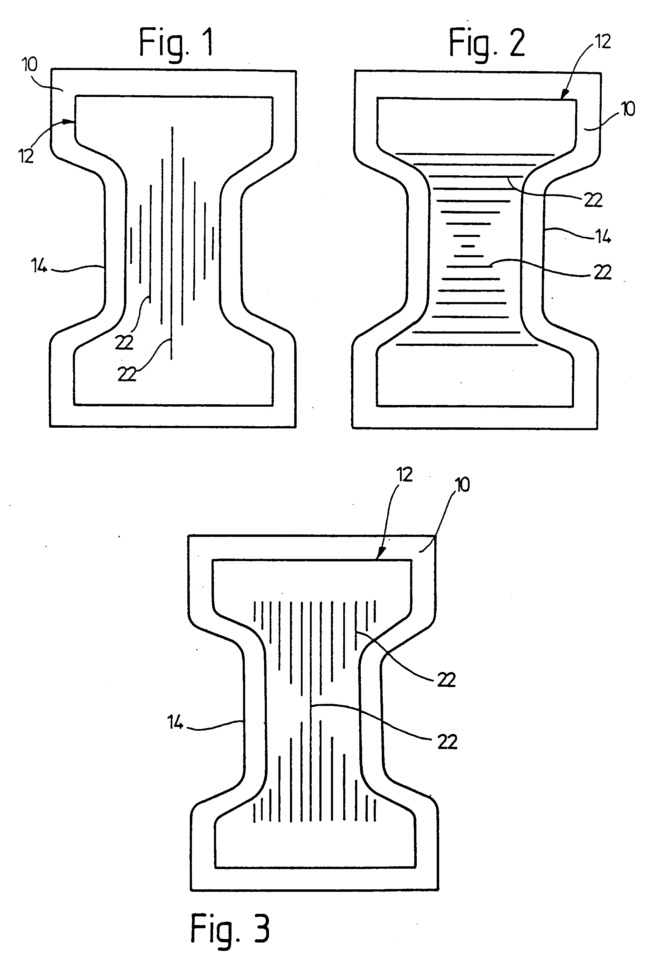

- Fig. 1 - 3:: jeweils eine Draufsicht auf die Aussenseite einer Wegwerfwindel mit gruppenweise unterschiedlich angeordneten Nässeanzeigestreifen.

Die in den Fig. 1 - 3 dargestellten Wegwerfwindeln weisen an ihrer einen Seite eine flüssigkeitsundurchlässige Wäscheschutzfolie 10 auf, die beispielsweise aus Polyäthylen besteht und zumindest durchscheinend ist.

Auf der Körperseite der Wäscheschutzfolie 10 ist ein Saugkörper 12 angeordnet, der aus einem geeigneten, flüssigkeitsabsorbierenden Material, z.B. einem Zellulosefasergemisch, geformt ist, dem vorzugsweise superabsorbierende Quellstoffsubstanzen beigemischt sind.

Der Saugkörper 12 kann im Schrittbereich 14 eine Verstärkung aufweisen, um beim Gebrauch der Windel anfallende Flüssigkeit möglichst schnell aufnehmen zu können und in diesem Bereich zu halten. Die Taillenbereiche des Saugkörpers 12 weisen demgegenüber eine geringere Dicke auf.

Die Körperseite des Saugkörpers 12 ist zweckmässigerweise von einer Abdeckschicht überzogen, die vorteilhafterweise aus einem porösen, flüssigkeitsdurchlässigen Vliesstoff gebildet wird. Diese Vliesstoffabdeckung ist in üblicher Weise im Randbereich der Windel mit der Wäscheschutzfolie 10 verbunden.

Auf der dem Saugkörper 12 zugewandten Seite der Wäscheschutzfolie 10 ist eine Vielzahl von Nässeanzeigestreifen 22 vorgesehen. Diese umfassen beispielsweise einen nässeempfindlichen Farbindikator, der mittels einer Trägersubstanz, z.B. einem thermoplastischen Material, insbesondere einem Heisskleber, auf die Körperseite der Wäscheschutzfolie 10 oder auf die dem Körper abgewandte Seite des Saugkörpers 12 aufgebracht ist. Der Farbindikator wechselt in Abhängigkeit von seinem trockenen oder nassen Zustand seine Farbe, so dass bei einer angelegten Wegwerfwindel von aussen erkannt werden kann, ob diese trocken oder nass ist und, wenn sie nass ist, in welchem Umfang die Wegwerfwindel noch Flüssigkeit aufzuehmen vermag.

Die Nässeanzeigemarkierungen müssen dabei entweder aus einem wasserunlöslichen Farbstoff oder aus einem Farbindikator gebildet werden, der erst bei Nässe deutlich sichtbar ist.

Als Nässeanzeigemittel eignen sich auch auswaschbare Farbindikatoren, die durch die vom Saugkörper aufgenommene Flüssigkeit ausgewaschen, also unsichtbar werden.

Fig. 1 zeigt dabei eine Anordnung von Nässeanzeigestreifen 22, die parallel zur Windellängsrichtung symmetrisch zu dieser angeordnet sind. Dabei nimmt die Länge der einzelnen Anzeigestreifen von der Windelmitte nach aussen hin ab, so dass die mittleren Anzeigestreifen 22 sich vom Schrittbereich 14 bis in die Taillenbereiche erstrecken, während die äussersten Anzeigestreifen 22 lediglich im Schrittbereich vorgesehen sind.

Fig. 2 zeigt eine Anordnung von Anzeigestreifen 22, die quer zur Windellängsrichtung vorgesehen sind und deren Länge vom Schrittbereich 14 zu den Taillenbereichen hin zunimmt.

Eine weitere, parallel zur Windellängsrichtung ausgerichtete Streifenanordnung ist in Fig. 3 dargestellt, wobei die einzelnen Nässeanzeigestreifen 22 eine sanduhrförmige Fläche markieren, die im wesentlichen symmetrisch zu der Stelle des grössten Flüssigkeitsanfalls im Schrittbereich 14 der Wegwerfwindel angeordnet ist.

Werden derartige Wegwerfwindeln bei der Pflege von inkontinenten Patienten oder anderen, pflegebedürftigen, inkontinenten Personen eingesetzt, so ist es für das Pflegepersonal ohne Öffnen der Windel einfach, von aussen zu erkennen, ob die Windel bereits so nass ist, dass sie gewechselt werden muss, dass also die Flüssigkeitsaufnahmekapazität erschöpft ist. Dabei ist die Anordnung der einzelnen Nässeanzeigestreifen 22 mit Abstand zueinander von Vorteil, da das Pflegepersonla so eindeutig erkennen kann, in welchen Windelbereichen bereits Körperflüssigkeit aufgenommen wurde und welche Bereiche noch zur Aufnahme von Flüssigkeit bereitstehen.

Auf diese Weise wird insbesondere auch vermieden, dass Wegwerfwindeln zu früh gewechselt werden, was zu einer Senkung der Pflegekosten führt, und umgekehrt wird vermieden, dass eine pflegebedürftige Person in einer nassen, mit Körperflüssigkeit überladenen Wegwerfwindel liegt.

## Patentansprüche

1. Wegwerfwindel mit einer Wascheschutzfolie (10), auf die körperseitig ein Saugkörper (12) aufgebracht ist, dem zwischen diesem und der Wäscheschutzfolie (10) angeordnete und im Abstand zueinander parallel verlaufende Nässeanzeigestreifen (22) zugeordnet sind, die zu einer Windelsymmetrieachse symmetrisch angeordnete, ohne Öffnen der angelegten Windel den eingenässten Bereich sichtbar machende Streifengruppen bilden,
**dadurch gekennzeichnet**,
dass die Nässeanzeigestreifen (22) beider Streifengruppen zu einer quer zur Windelsymmetrieachse liegenden weiteren Windelsymmetrieachse unterschiedlich lang und in bezug auf die dem grössten Flüssigkeitsanfall zugeordnete Stelle des Saugkörpers (12) symmetrisch angeordnet sind.

2. Wegwerfwindel nach Anspruch 1, dadurch gekennzeichnet, dass zwischen beiden Streifengruppen zumindest ein auf der Windelsymmetrieachse liegender mittlerer Nässeanzeigestreifen (22) vorgesehen ist.

3. Wegwerfwindel nach Anspruch 1 oder 2, dadurch gekennzeichnet, dass die Nässeanzeigestreifen (22) sich in Windellängsrichtung erstrecken und sich deren Länge je Streifengruppe in Richtung auf den jeweiligen Windellängsrand verringert.

4. Wegwerfwindel nach Anspruch 1 oder 2, dadurch gekennzeichnet, dass die Nässeanzeigestreifen (22) sich in Windelquerrichtung erstrecken und sich deren Länge je Streifengruppe in Richtung auf jeweils eines der Windelenden vergrössert.

5. Wegwerfwindel nach einem der vorhergehenden Ansprüche, dadurch gekennzeichnet, dass die Länge jedes Nässeanzeigestreifens (22) je Streifengruppe zu der in Streifenquerrichtung liegenden Windelsymmetrieachse symmetrisch veränderbar ist.

## Claims

1. Disposable diaper having a protective film (10) carrying on its surface adjacent the body an absorbent body (12) which has associated to it wetness indicator strips (22), which are provided between the absorbent body (12) and the protective film (10), in spaced and mutually parallel arrangement, and which form groups of strips that are distributed symmetrically relative to an axis of symmetry of the diaper and make the wetted area visible without the need to open the applied diaper,
**characterized in that**
the wetness indicator strips (22) of both groups of strips are different in length, relative to another axis of symmetry of the diaper that extends crosswise to the said axis of symmetry of the diaper, and are arranged symmetrically relative to that point of the absorbent body (12) that is associated to the area where the greatest amount of liquid is expected to occur.

2. Disposable diaper according to claim 1, characterized in that there is provided between said two groups of strips at least one central wetness indicator strip (22) that coincides with the axis of symmetry of the diaper.

3. Disposable diaper according to claim 1 or 2, characterized in that said wetness indicator strips (22) extend in the longitudinal direction of the diaper and that their length increases in each group of strips toward the respective longitudinal edge of the diaper.

4. Disposable diaper according to claim 1 or 2, characterized in that said wetness indicator strips (22) extend in crosswise direction of the diaper, and that their length increases in each group of strips toward one of the ends of the diaper.

5. Disposable diaper according to any of the preceding claims, characterized in that the length of each said wetness indicator strip (22) in each said group of strips can be varied symmetrically relative to the axis of symmetry of the diaper extending in crosswise direction of the strips.

## Revendications

1. Lange à jeter avec une feuille de protection de linge (10), sur laquelle est appliqué côté corps un corps absorbant (12) auquel sont coordonnées des bandes indicatrices d'humidité (22) disposées entre le corps absorbant (12) et la feuille de protection (10) et parallèles l'une à l'autre, ces bandes formant des groupes symétriques à un axe de symétrie de la lange et rendant visible la zone humide sans devoir ouvrir la lange appliquée,
caractérisée en ce que
les bandes indicatrices d'humidité (22) des deux groupes de bandes sont disposées avec différentes longueurs par rapport à un autre axe de symétrie de la lange transversal à l'axe de symétrie de la ange et symétriques à l'endroit du corps absorbant (12) coordonné au côté du corps absorbant avec la plus grande production d'humidité.

2. Lange à jeter selon la revendication 1, caractérisée en ce que entre les deux groupes de bandes est prévue au moins une bande médiane indicatrice d'humidité (22) située sur l'axe de symétrie de la lange.

3. Lange à jeter selon la revendication 1 ou 2, caractérisée en ce que les bandes indicatrices d'humidité (22) s'étendent en direction longitudinale de la lange et que en fonction du groupe de bandes leur longueur se réduit en direction du bord longitudinal de la lange.

4. Lange à jeter selon la revendication 1 ou 2, caractérisée en ce que les bandes indicatrices d'humidité (22) s'étendent en direction transversale à la lange et que en fonction du groupe de bandes leur longueur grandit en direction d'une des extrémités respectives de la lange.

5. Lange à jeter selon l'une des revendications précédentes, caractérisée en ce que la longueur de chaque bande indicatrice d'humidité (22) est en fonction du groupe de bande modifiable symétriquement par rapport à l'axe de symétrie de la lange situé en sens transversal des bandes.
